# EUROPEAN PATENT APPLICATION

(11) **EP 1 980 236 A1**
(43) Date of publication of application: **15.10.2008**
(21) Application number: 07706450.9
(22) Date of filing: 09.01.2007
(51) Int. Cl.: A61K 8/19, A61Q 19/02

(54) **COSMETIC**

(30) Priority: 06.01.2006 JP 2006001857
(71) Applicant: Atom Japan, Inc., Tsu-shi, Mie 5142221 (JP)
(72) Inventor: HIROBE, Tomohisa, Chiba-shi, Chiba 2620026 (JP); AKATSUKA, Koichi, Tsu-shi, Mie 5142293 (JP)
(74) Representative: Strehl Schübel-Hopf & Partner
(86) International application number: PCT/JP2007/050103
(87) International publication number: WO 2007/078009

(57) **Abstract**

A cosmetic preparation comprising an aqueous solution of a water-soluble iron salt dimer, said aqueous solution containing magnesium ion and calcium ion.

## Description

### FIELD OF THE INVENTION

The present invention relates to a cosmetic preparation acting on keratinocytes and melanocytes affecting sunburn, stains and freckles to exhibit excellent cosmetic effect.

### BACKGROUND OF THE INVENTION

Erythema generated on the skin by ultraviolet irradiation is turned black as so-called sunburn. Excessive ultraviolet irradiation is likely to provide blisters like burn. Continuous ultraviolet irradiation appears to accelerate skin aging, and form stains and freckles. Though their mechanisms are not clear yet, it is considered that stains and freckles are generated by the activation of melanocytes in the epidermal basal layer with ultraviolet rays, causing excessive production of melanin pigments.

Consumers have become increasingly interested in cosmetics having a so-called "whitening effect" of removing stains and freckles, and various whitening cosmetics have been commercially available so far. Components conventionally used in whitening cosmetics include peroxides such as hydrogen peroxide, zinc peroxide, magnesium peroxide, etc., and various natural substances such as ascorbic acid. However, ascorbic acid has poor stability and durability, etc., and its effect is not necessarily sufficient. Though hydroquinone is used as a skin-whitening agent in the U.S., etc., it is undesirable as an ingredient in cosmetics from the aspect of safety, stimulus, allergy, etc. Accordingly, various cosmetics having skin-whitening effects without any problem of safety, etc. have been investigated.

Proposed as safer cosmetics are those containing kojic acid and its derivatives, or hydroquinone glycoside and its derivatives as an effective component. Although the effectiveness of cosmetics containing these components has been observed on a cell level, it is insufficient on a human level.

JP 9-241145 A discloses a cosmetic composition comprising an Ginkgopsida leave extract formulated in an aqueous solution containing trace amounts of bivalent iron and trivalent iron. JP 9-241145 A describes that the diluted bivalent/trivalent irons and the Ginkgopsida leave extract penetrate into the skin cells to make the skin look fresh, and also into the blood vessels to strengthen the phagocytosis of neutrophils in leukocytes, thereby enhancing immunity. However, this cosmetic composition has substantially no whitening effect.

### OBJECT OF THE INVENTION

Accordingly, an object of the present invention is to provide a cosmetic preparation exhibiting an excellent whitening effect while being completely harmless to the health.

### SUMMARY OF THE INVENTION

As a result of intensive research in view of the above object, the inventors have found that a cosmetic preparation based on an aqueous solution of a water-soluble iron salt dimer and containing magnesium ion and calcium ion accelerates the proliferation and differentiation of keratinocytes and melanocytes, thereby exhibiting excellent cosmetic effect. The present invention has been completed based on such finding.

Thus, the cosmetic preparation of the present invention is based on an aqueous solution of a water-soluble iron salt dimer, and further contains magnesium ion and calcium ion.

The amount of the water-soluble iron salt dimer is preferably 0.01-10000 µg/L. The concentration of magnesium is preferably 0.01-5000 mg/L. The concentration of calcium is preferably 1-10000 mg/L.

### BRIEF DESCRIPTION OF THE DRAWING

Fig. 1 is a graph showing the number of cells before and after the culture in Reference Example 1(i).

Fig. 2 is a graph showing a differentiation rate in Reference Example 1 (i).

Fig. 3 is a graph showing the number of cells before and after the culture in Reference Example 1(ii).

Fig. 4 is a graph showing a differentiation rate in Reference Example 1 (ii).

Fig. 5 is a graph showing the number of cells before and after the culture in Reference Example 1(iii).

Fig. 6 is a graph showing a differentiation rate in Reference Example 1 (iii).

Fig. 7 is a graph showing the number of cells before and after the culture in Reference Example 1(iv).

Fig. 8 is a graph showing a differentiation rate in Reference Example 1 (is).

Fig. 9 is a graph showing the number of cells before and after the culture in Reference Example 2(i).

Fig. 10 is a graph showing a differentiation rate in Reference Example 2(i).

Fig. 11 is a graph showing the number of cells before and after the culture in Reference Example 2(ii).

Fig. 12 is a graph showing a differentiation rate in Reference Example 2(ii).

Fig. 13 is a graph showing the number of cells before and after the culture in Reference Example 2(iii).

Fig. 14 is a graph showing a differentiation rate in Reference Example 2(iii).

Fig. 15 is a graph showing the mitotic index of melanocytes in Reference Example 2(iv).

Fig. 16 is a graph showing the number of cells before and after the culture in Reference Example 2(iv).

Fig. 17 is a graph showing a differentiation rate in Reference Example 2(iv).

Fig. 18 is a graph showing the mitotic index of melanoblasts in Reference Example 2(iv).

Fig. 19 is a graph showing the number of cells before and after the culture in Comparative Example 1(i).

Fig. 20 is a graph showing a differentiation rate in Comparative Example 1(i).

Fig. 21 is a graph showing the number of cells before and after the culture in Comparative Example 1(ii).

Fig. 22 is a graph showing a differentiation rate in Comparative Example 1(ii).

### DETAILED DESCRIPTION OF THE PREFERRED EMBODIMENTS

[1] Cosmetic preparation

The cosmetic preparation of the present invention contains magnesium ion and calcium ion in an aqueous solution of a water-soluble iron salt dimer. The term "cosmetic preparation" used herein includes not only a basic cosmetic preparation such as cosmetic water, emulsion, cream and liquid, and lip cream, but also a makeup cosmetic preparation such as foundation, lipstick and eyeshadow. Components contained in the cosmetic preparation will be explained below.

(1) Water-soluble iron salt dimer

The water-soluble iron salt dimer (ferrous/ferric chloride) is a composite comprising bivalent iron ion and trivalent iron ion, typically having a composition represented by Fe₂Cl₅. An aqueous solution of the water-soluble iron salt dimer has large surface tension and osmotic pressure, accelerating the proliferation and differentiation of keratinocytes, melanoblasts and melanocytes in the epidermis, thereby exhibiting a whitening effect, and strengthening the phagocytosis of neutrophils in the leukocytes to enhance immunity and to prevent aging. The bivalent iron ions to the trivalent iron ions obtained by dissolving the water-soluble iron salt dimer have different ratios depending on the conditions of synthesizing the water-soluble iron salt dimer, and both iron ions can exist at arbitrary percentages. Both ions are called "bivalent/trivalent iron ions" as a whole.

The concentration of the bivalent/trivalent iron ions (expressed by the amount of the water-soluble iron salt dimer) in the cosmetic preparation is preferably 0.01-10000 µg/L, more preferably 0.1-1000 µg/L, particularly 1-500 µg/L. When the concentration of the bivalent/trivalent iron ions is less than 0.01 µg/L, only a small whitening effect is obtained when the cosmetic preparation is applied. When the concentration of the bivalent/trivalent iron ions exceeds 10000 µg/L, an effect of accelerating the proliferation and differentiation of melanoblasts, etc. is saturated, with further increase not expected.

The cultivation of skin cells of an infant mouse in a medium containing an aqueous solution of a water-soluble iron salt dimer, calcium ion, and magnesium ion has revealed that the proliferation of keratinocytes, melanoblasts and melanocytes is accelerated 2-2.5 times as much as when the skin cells are cultivated in a medium not containing this aqueous solution. It may thus be presumed that this aqueous solution has effects of preventing and/or reducing stains and freckles and whitening the skin.

(2) Calcium ion

The concentration of calcium ion in the cosmetic preparation is preferably 0.1-10000 mg/L, more preferably 1-5000 mg/L. When the concentration of calcium ion is less than 1 mg/L, the cosmetic preparation does not effectively act on melanoblasts and melanocytes, failing to exhibit a sufficient whitening effect. However, when the concentration of calcium ion exceeds 10000 mg/L, the proliferation of melanoblasts and melanocytes is rather hindered. The concentration of calcium ion is particularly preferably on the same level as in a Ham's F-10 medium (available from GIBCO, etc.) generally used as a culture medium for skin cells. With the concentration of magnesium ion (described later) and the concentration of calcium ion substantially on the same level as in the Ham's F-10 medium, the proliferation and differentiation of melanoblasts and melanocytes are accelerated, resulting in an excellent whitening effect. The molar ratio of the bivalent/trivalent iron ions to the calcium ion (water-soluble iron salt dimer /calcium) is preferably 1-10000, more preferably 10 to 5000, particularly 100 to 1000. Inorganic salts for forming the calcium ion are not restrictive as long as they are harmless to the skin.

(3) Magnesium ion

The concentration of magnesium ion in the cosmetic preparation is preferably 0.01-5000 mg/L, more preferably 0.05-500 mg/L. When the concentration of magnesium ion is less than 0.01 mg/L, the cosmetic preparation does not effectively act on melanoblasts and melanocytes, failing to exhibit a sufficient whitening effect. However, when the concentration of magnesium ion exceeds 5000 mg/L, the proliferation of melanoblasts and melanocytes is hindered. Like the concentration of calcium ion, the concentration of magnesium ion is particularly preferably on the same level as in the Ham's F-10 medium. Inorganic salts for forming the magnesium ion are not restrictive as long as they are harmless to the skin.

The molar ratio of the calcium ion to the magnesium ion is preferably 1/10 to 10. The molar ratio of the bivalent/trivalent iron ions to the magnesium ion (water-soluble iron salt dimer/magnesium ion) is preferably 1-100000, more preferably 10 to 10000, particularly 100 to 1000. The molar ratio of the calcium ion to the magnesium ion and the molar ratio of the bivalent/trivalent iron ions to the magnesium ion in these ranges provide a large effect of proliferating melanocytes, etc., exhibiting an extremely large whitening effect.

(4) Optional components

Optional components that may be contained in the cosmetic preparation of the present invention include components contained in the cell medium (for instance, Ham's F-10), such as inorganic salts, amino acids, adenine, ethanolamine, phosphoethanolamine, phenol red Na, putrescine 2HCl, thiamine HCl, thioctic acid, thymidine, glucose, HEPES and antibiotics; components formulated in usual cosmetics such as oils, surfactants, humectants, lower alcohols, thickeners, antioxidants, chelating agents, pH-adjusting agents, antiseptics, fragrances, pigments, ultraviolet absorbers, ultraviolet-scattering agents and vitamins; natural drugs, proteolytic enzymes, etc. If the cosmetic preparation had a composition closer to that of the Ham's F-10 medium, the proliferation and differentiation of melanoblasts and melanocytes would be further accelerated. The concentration of the optional components is preferably about 1/100 to 100 times, more preferably 1/10 to 10 times, as much as in the Ham's F-10.

Examples of inorganic salts other than the water-soluble iron salt dimers, calcium salts and magnesium salts include sodium chloride, sodium carbonate, sodium hydrogen carbonate, sodium sesquicarbonate, sodium sulfate, sodium sulfite, sodium silicate, sodium phosphate, sodium dibasic phosphate, sodium polyphosphate, sodium hydrogen phosphate, sodium selenite, sodium acetate, sodium pyruvate, potassium chloride, potassium sulfate, potassium dihydrogen phosphate, potassium iodide, ammonium chloride, anhydrous silicic acid, metasilicic acid, copper (II) sulfate, iron (III) sulfate, manganese chloride, nickel sulfate, tin chloride, zinc sulfate, aluminum sulfate, aluminum silicate, borax, ammonium metavanadate, ammonium molybdenate, etc.

Examples of the amino acids include L-alanine, L-arginine, L-asparagine, L-aspartic acid, L-cysteine, L-cystine, L-glutaminic acid, glutamine, glycine, L-histidine, L-isoleucine, L-leucine, L-ricin, L-methionine, L-phenylalanine, L-proline, L-hydroxyproline, L-serine, L-threonine, L-tryptophan, L-tyrosine, L-valine, etc.

The oils include essential oils such as mint oil, jasmine oil, camphor oil, thuja oil, spruce oil, creosote oil, turpentine oil, cinnamon oil, bergamot oil, orange oil, sweet flag oil, pine oil, lavender oil, bay oil, clove oil, cedar oil, rose oil, eucalyptus oil, lemon oil, thyme oil, peppermint oil, rose oil, sage oil, menthol, cineol, eugenol, citral, citronellal, borneol, linalool, geraniol, camphor, thymol, spilanthol, pinene, and limonene; fats and oils such as olive oil, jojoba oil, castor oil, coconut butter, camellia oil, coconut oil, tree wax, grape seed oil, avocado oil, mink oil, egg yolk oil, and hardened oil; waxes such as whale wax, honey wax, lanoline, carnauba wax, and candelilla wax; hydrocarbons such as liquid paraffin, ceresin, squalene, microcrystalline wax, paraffin wax, and vaseline; aliphatic acids such as stearic acid, oleic acid, lauric acid, myristic acid, isostearic acid, palmitic acid, and behenic acid; higher alcohols such as cetyl alcohol, stearyl alcohol, lanolin alcohol, octyldodecanol, and hexyl decanol; esters such as isopropyl myristate, butyl stearate, isopropyl palmitate, octyldodecyl myristate, and cholesterol oleate.

The surfactants include (a) anionic surfactants such as sodium stearate, cetyl sodium sulfate, polyoxyethylene lauryl ether phosphate, sodium lauryl phosphate, triethanolamine palmitate, sodium N-acyl glutamate, etc., (b) cationic surfactants such as stearyl dimethyl benzyl ammonium chloride, stearyl trimethyl ammonium chloride, etc., (c) amphoteric surfactants such as chloroalkylamino ethyleneglycine liquid, lecithin, etc., nonionic surfactants such as glyceryl monostearate, sorbitan monostearate, sucrose-fatty acid esters, propylene glycol monostearate, polyoxyethylene oleyl ether, polyethylene glycol monostearate, polyoxyethylene sorbitan monopalmitate, polyoxyethylene coconut acid monoethanolamide, polyoxyethylene polyoxypropylene glycol, polyoxyethylene castor oil, polyoxyethylene lanoline, etc.

The humectants include polyvalent alcohols such as glycerin, 1,3-butylene glycol, propylene glycol, sorbitol, polyethylene glycol, and dipropylene glycol; NMF components such as amino acids, sodium lactate, and pyrrolidone sodium carboxylate; water-soluble, high-molecular materials such as hyaluronic acid, collagen, mucopolysaccharides, and chondroitin sulfate; etc.

The lower alcohols include ethanol and isopropyl alcohol.

The thickeners include sodium alginate, xanthan gum, cydonia oblonga extracts, tragacanth gum, starch, casein, water-soluble gelatin, pectin, karaya gum, locust bean gum, carrageenan, carbopol, acacia gum, agar, methyl cellulose, hydroxyethyl cellulose, carboxymethylcellulose, cationic cellulose, polyvinylpyrrolidone, polyvinyl methyl ether, carboxyvinyl polymers, polyvinyl alcohol, etc.

The antioxidants include dibutyl hydroxytoluene, butylhydroxyanisole, propyl gallate, and ascorbic acid. The chelating agents include disodium edetate, ethane hydroxy diphosphate, pyrophosphate, hexametaphosphate, citric acid, tartaric acid, and glucuronic acid. The pH-adjusting agents include sodium hydroxide, triethanolamine, citric acid, sodium citrate, boric acid, and sodium hydrogen phosphate. The antiseptics include methyl p-hydroxybenzoate, dehydroacetic acid, salicylic acid, benzoic acid, sorbic acid, and benzalkonium chloride. The ultraviolet absorbers include 2-hydroxy-4-methoxybenzophenone, octyl dimethyl p-aminobenzoate, and ethyl hexyl p-methoxy cinnamate. The ultraviolet-scattering agents include titanium oxide, kaolin, talc, etc. The vitamins include vitamin A, vitamin B, vitamin C, vitamin D, vitamin E, vitamins F, vitamin K, vitamin P, vitamin U, carnitine, ferulic acid, γ-oryzanol, α-lipoic acid, orotic acid and derivatives thereof.

The natural drugs include *Atractylodis lanceae rhizoma, Atractylodis rhizoma, Vlerian officinale, Sizonepetae spica, Mgnoliae cortex, Cnidium officinale,* bitter orange peel, *Angelica acutiloba, Zingiber officinale, Scutellaria baicalensis, Gardenia, Artemisiae folium*, aloe, carrot, cinnamon, peony root, mentha arvensis leaf, *Poria cocos, acous calamus, Schisandra nigra Maxmim*, juniper, saffron, *Phellodendri cortex,* fennel, citrus unshiu peel, *Diplomorpha sikoki*, chamomile, radish, willow, camphor tree, *Sambucus nigra, Sambucus chinensis, Elsholtzia ciliata*, fatsia, *Acorus gramineus*, mogwort, *Hypericum perforatum*, citrus, *Citrus aurantium,* peach, *Gleditsia japonica*, loquat, *Lonicera japonica, Angelica dahurica*, linden tree, horse chestnut, yarrow, hop, rosemary, *Fuscoporia obliqua*, pine, Yarenzura, lantana, licorice, *Valeriana officinalis*, horse chestnut, etc.

[2] Production method of cosmetic preparation

The water-soluble iron salt dimer can be produced, for instance, by a synthesis method described in "Production Method of Water-Soluble Iron Salt Dimer," Jiro Sugi, et. al., Resume of Lectures of Research and Technology in Annual Meeting of The Oceanographic Society of Japan, Vol. 42nd, p. 11 (1991), which will be explained below.

A mixture liquid of 2 mol of ammonium formate, 1 mol of hydroxylamine and 1 mol of formamide is mixed with 1 mol of ferric chloride (FeCl₃-6H₂O). This preparation liquid is successively diluted with distilled water to obtain a liquid α (10⁻⁸ mM), a liquid β (10⁻¹² mM) and a liquid γ (10⁻¹⁴ mM). 1 g/10 mL of ferric chloride (FeCl₃-6H₂O) is dissolved in each of the resultant liquids α, β and γ, slowly dried by evaporation at 100°C or lower to obtain iron chloride crystals α, β and γ. Measurement by a Mössbauer method reveals that the ratio of bivalent iron to trivalent iron in bivalent/trivalent iron salts obtained by this synthesis method is 4/6 in the crystal α, 6/4 in the crystal β, and 7/3 in the crystal γ. The above reference describes that it is presumed by ion chromatography, X-ray analysis, etc. that bivalent iron and trivalent iron in the iron chloride are not in a simple mixture but in the form of a dimer. The bivalent/trivalent iron salts used in the present invention are not restricted to those obtained by the above synthesis method, and the bivalent/trivalent iron ratio is not restricted to the above values.

Among commercially available drinks, etc., those containing the water-soluble iron salt dimer are used as cosmetic materials. Their examples are drinks commercially available under the name of Pairogen (registered trademark of Yugen Kaisha Atom Japan, and sold by Akatsuka Co., Ltd.), and water in which FFC ceramics are immersed ("FFC" is a registered trademark of Yugen Kaisha Atom Japan, and sold by FFC Japan Co., Ltd.). When about 10 to 100 FFC ceramic balls (25-250 cm³) are immersed in 1 L of water at room temperature for 3-48 hours, an aqueous solution of a water-soluble iron salt dimer can be obtained at the above preferable concentration. Pairogen and FFC ceramics-immersed water are particularly preferable, because they contain an aqueous solution of a water-soluble iron salt dimer, calcium ion and magnesium ion in the above preferable concentrations, and because they quickly penetrate into the skin by application, exhibiting an effect of activating the proliferation of keratinocytes, melanoblasts and melanocytes.

The inventors' research has revealed that cosmetic water using Pairogen or the FFC ceramics-immersed water as an ingredient exhibits an excellent whitening effect. Such effect can be obtained, presumably because Pairogen and FFC ceramics-immersed water contain various elements such as copper, molybdenum, zinc, strontium, barium, vanadium, boron, etc., which exhibit a synergistic effect with indispensable elements such as the aqueous solution of the water-soluble iron salt dimer. It is thus considered that the proliferation and differentiation of keratinocytes, melanoblasts or melanocytes are further accelerated in the cosmetic preparation containing Pairogen or the FFC ceramics-immersed water. The preferred concentrations by mass of these elements are 0.001-100 ppb of copper, 0.001-100 ppb of molybdenum, 0.001-100 ppb of zinc, 0.001-1 ppm of strontium, 0.0001-1 ppm of barium, 0.0001-0.1 ppm of vanadium, and 0.001-100 ppb of boron.

Although Pairogen and the FFC ceramics-immersed water contain calcium ion and magnesium ion as described above, these metals may be added so that they have compositions equal to or close to that of the Ham's F-10 medium. With the composition equal to or close to that of the Ham's F-10 medium, the cosmetic preparation is provided with a further improved whitening effect. Salts containing calcium, etc. may be added to Pairogen or the FFC ceramics-immersed water, or the aqueous solution of the water-soluble iron salt dimer may be added to a cream in which calcium, etc. are dispersed. Namely, they may be used in a desired state depending on the type of cosmetics produced (cosmetic water, emulsion, cream, etc.).

The cosmetic preparation containing the aqueous solution of a water-soluble iron salt dimer, calcium and magnesium acts on keratinocytes, melanoblasts and melanocytes, accelerating their proliferation and differentiation. The inventors' research has revealed that the proliferation of keratinocytes, melanoblasts and melanocytes accelerated to 2-2.5 times in a solution containing the aqueous solution of a water-soluble iron salt dimer, as well as calcium ion and magnesium ion in substantially the same concentration as in the Ham's F-10 medium. If any one of the aqueous solution of a water-soluble iron salt dimer, the calcium ion and the magnesium ion were missing, such effect would not be obtained. For instance, in a solution not containing calcium or magnesium, keratinocytes, melanoblasts and melanocytes cannot be cultured. In a solution not containing the aqueous solution of a water-soluble iron salt dimer, culture is possible, but the proliferation and differentiation of keratinocytes, melanoblasts and melanocytes cannot be accelerated. Accordingly, a cosmetic preparation not containing any one of the aqueous solution of a water-soluble iron salt dimer, calcium ion and magnesium ion does not have a sufficient whitening effect.

The present invention will be described in detail referring to Examples below without intention of restricting the present invention thereto.

Example 1

(i) Preparation of FFC water

40 FFC ceramic balls of about 16 mm in diameter were washed with ultra-pure water for 2 minutes, immersed in 900 mL of ultra-pure water for 24 hours, and filtered with a glass fiber filter (1 µm). The resultant aqueous solution was measured with respect to the concentrations of bivalent/trivalent iron ions, calcium ion and magnesium ion. The results are shown in Table 1.

**Table 1**

| Ions | Concentration |
|---|---|
| Calcium | 25 mg/L |
| Magnesium | 2.6 mg/L |
| Iron | 0.074 ppm* |

| | |
|---|---|
| Note: * by mass. | |

(ii) Clinical experiment

60 g of FFC ceramic balls of about 16 mm in diameter were immersed in 2 L of deionized and distilled water (DDW) for 8 hours to prepare FFC water. The resultant FFC water, or Pairogen (a drink comprising a water-soluble iron salt dimer, high-fructose corn syrup, soya-oligosaccharide, honey, apple juice, rice vinegar, apple vinegar, persimmon vinegar, plum vinegar, citric acid, malic acid, plum extract, stevia, vitamin C, vitamin B₂, vitamin B₆, fragrance, and natural mineral water), which was diluted with the FFC water about 10 to 100 times, was applied to subject' faces about 1-3 times a day for 3 months, to compare skin colors before the experiment and 3 months after the experiment. The results are shown in Table 2.

**Table 2**

| Subjects | | Number of Subjects | | | | |
|---|---|---|---|---|---|---|
| | | Very Whitened | Whitened | Slightly Whitened | Substantially Not Changed | Total |
| Male | 40s | - | - | 1 | - | 1 |
| | 50s | 2 | - | 1 | 2 | 5 |
| | 60s | 1 | - | 2 | - | 3 |
| Female | 20s | - | 2 | - | - | 2 |
| | 30s | - | 1 | - | - | 1 |
| | 40s | 1 | - | 1 | - | 2 |
| | 50s | 1 | 1 | 1 | 1 | 4 |
| | 60s | - | 3 | 1 | 1 | 5 |
| Total | | 5 | 7 | 7 | 4 | 23 |

As is clear from Table 2, the whitening effect was appreciated in 19 subjects among 23, as high as 82.6% effective.

Reference Example 1

Using a serum-free culture method of primary epidermal cells (mainly consisting of keratinocytes and melanocytes) developed by the inventors, the following investigation was conducted to examine the function of Pairogen containing an aqueous solution of a water-soluble iron salt dimer to animal skin, particularly to keratinocytes and melanocytes.

(i) MDM + Pairogen

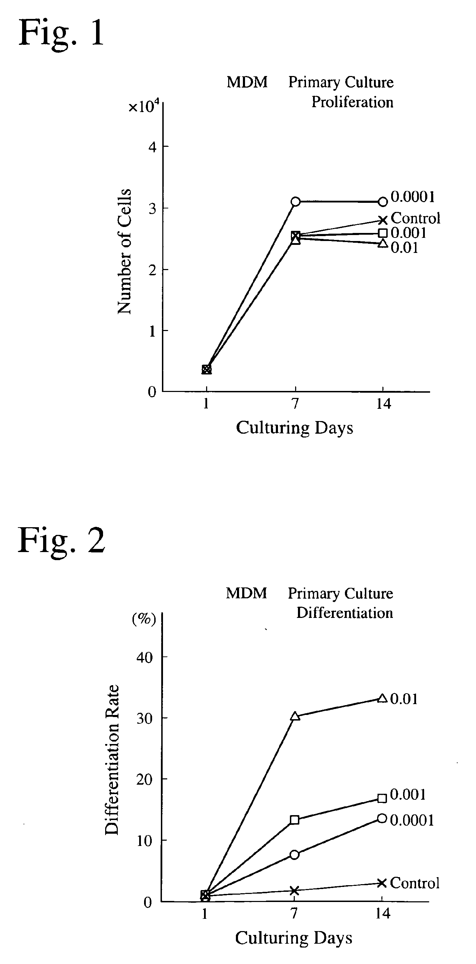

Pairogen was added to a melanoblast-defined medium (MDM) for purely culturing melanoblasts, origin of melanocytes, and used for the culture of primary epidermal cells of black inbred mice of 0.5 days after birth, whose skin was not yet black. The melanoblast-defined medium (MDM) was prepared by adding 10 µg/mL of insulin, 0.5 mg/mL of bovine serum albumin, 1 µM of ethanolamine, 1 µM of phosphoethanolamine and 1nM of sodium selenite to a Ham's F-10 medium (available from GIBCO). In the medium sample containing MDM and Pairogen, Pairogen was diluted 100 times, 1000 times, and 10000 times, respectively. Samples with any Pairogen concentrations had larger keratinocyte colonies with increased numbers of keratinocytes than a control. The number of keratinocytes in each sample was 2-2.5 times as many as in the control. The measured numbers and differentiation rates of melanoblasts and melanocytes are shown in Figs. 1 and 2. In the media containing Pairogen, larger numbers of melanin-forming melanocytes appeared. The effect of accelerating the proliferation of melanocytes was largest in the medium with 100-times-diluted Pairogen, and became smaller as diluted to 1000 times and 10000 times (Fig. 2). With respect to the number of cells, there was no effect provided by the addition of Pairogen (Fig. 1).

(ii) MDM+ Pairogen + melanocyte-stimulating hormone (MSH)

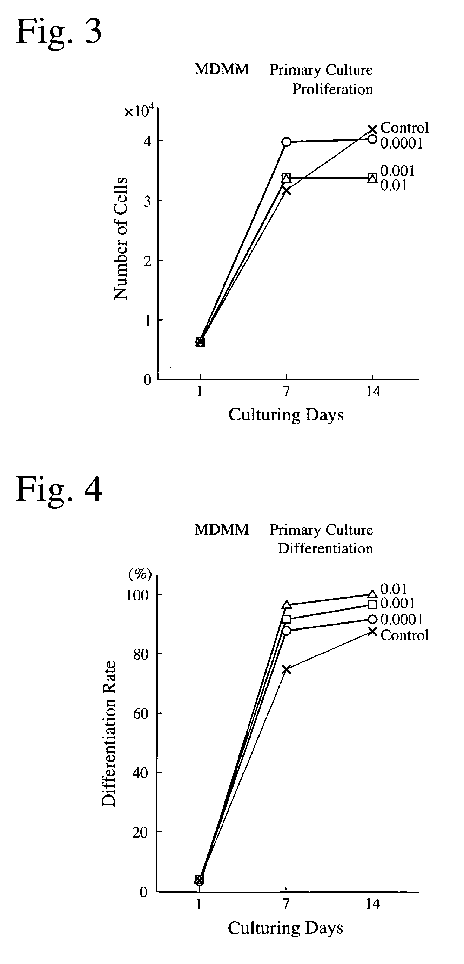

Primary mouse epidermal cells were cultured in the same manner as in Reference Example 1(i), except that Pairogen and a melanocyte-stimulating hormone (MSH) were added to MDM. Samples with any Pairogen concentrations had larger keratinocyte colonies with increased numbers of keratinocytes than a control. The number of keratinocytes in each sample was 2-2.5 times as many as in the control. The measured numbers and differentiation rates of melanoblasts and melanocytes are shown in Figs. 3 and 4. Melanocytes grew faster with larger dendrites and more melanin in the media containing Pairogen than in the Pairogen-free medium. The effect of accelerating the proliferation of melanocytes was largest in the medium with 100-times-diluted Pairogen, and became smaller as diluted to 1000 times and 10000 times (Fig. 4). With respect to the number of cells, there was no effect provided by the addition of Pairogen (Fig. 3).

(iii) MDM + Pairogen + dibutyryl cyclic adenosine monophosphate (DBcAMP)

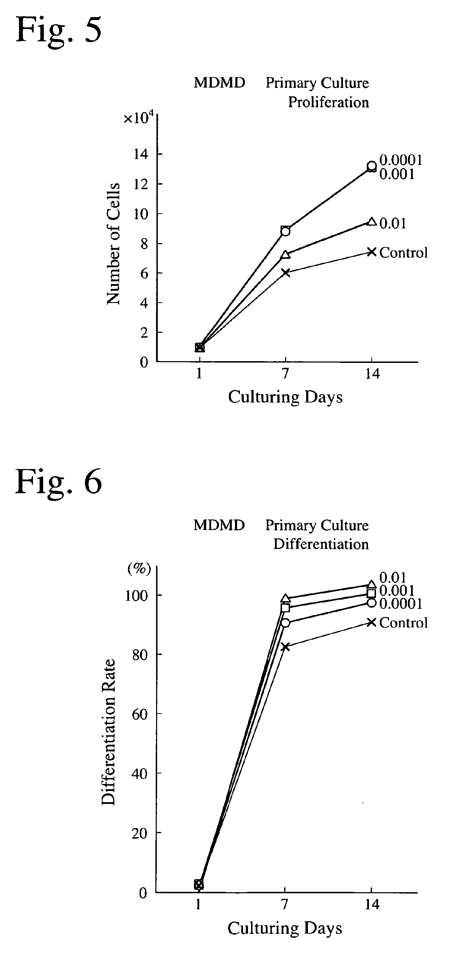

Primary mouse epidermal cells were cultured in the same manner as in Reference Example 1(i), except that Pairogen and dibutyryl cyclic adenosine monophosphate (DBcAMP) important for the proliferation of melanocytes were added to MDM. Samples with any Pairogen concentrations had larger keratinocyte colonies with increased numbers of keratinocytes than a control. The number of keratinocytes in each sample was 2-2.5 times as many as in the control. The numbers of melanoblasts and melanocytes were measured to determine their differentiation rates. The results are shown in Figs. 5 and 6. Keratinocytes and melanocytes grew faster with larger dendrites to provide increased melanin and larger cells in the media containing Pairogen than in the Pairogen-free medium. Cells started proliferating after 3-4 days, and after 14 days they became about 2 times as many as in the Pairogen-free medium. The effect of accelerating the proliferation of melanocytes was largest in the medium with 100-times-diluted Pairogen, and became smaller as diluted to 1000 times and 10000 times (Fig. 6). The function of increasing melanocytes was largest when Pairogen was as thinnest as 10000 times (Fig. 5).

(iv) MDM+ Pairogen + DBcAMP+ basic fibroblast growth factor (bFGF)

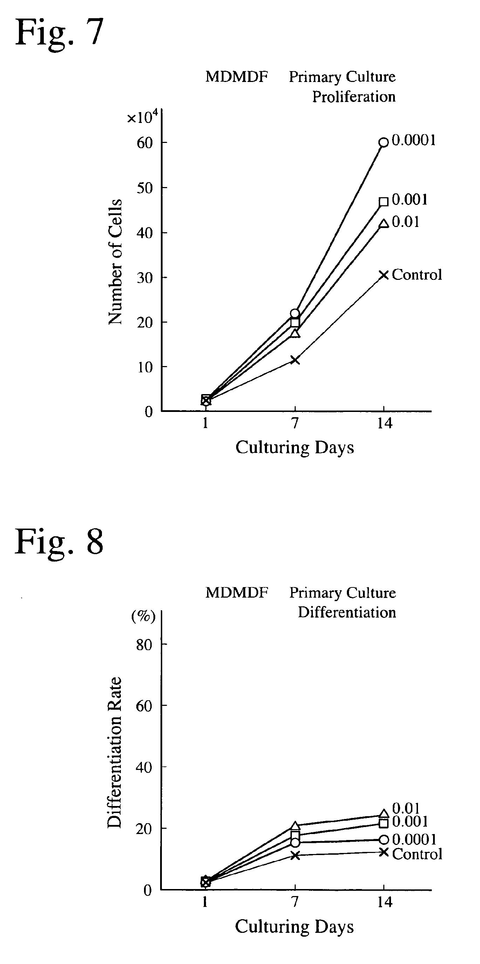

Primary mouse epidermal cells were cultured in the same manner as in Reference Example 1(i), except that Pairogen, melanoblast-increasing DBcAMP and a basic fibroblast growth factor (bFGF) were added to MDM. Samples with any Pairogen concentrations had larger keratinocyte colonies with increased numbers of keratinocytes than a control. The number of keratinocytes in each sample was 2-2.5 times as many as in the control. The numbers of melanoblasts and melanocytes were measured to determine their differentiation rates. The results are shown in Figs. 7 and 8. Keratinocytes grew faster in the media containing Pairogen than in the Pairogen-free medium. Melanoblasts started proliferating after 3-4 days, and after 14 days they became about 2 times as many as in the Pairogen-free medium. The addition of Pairogen increased the percentage of melanocytes. The effect of accelerating the proliferation of melanocytes was largest in the medium with 100-times-diluted Pairogen, and became smaller as diluted to 1000 times and 10000 times (Fig. 8). The function of increasing melanocytes was largest when Pairogen was as thinnest as 10000 times, and decreased when Pairogen became thicker to 1000 times and 100 times (Fig. 7).

Reference Example 2

60 g of FFC ceramics were immersed in 2 L of deionized and distilled water (DDW) for 8 hours, and filtered with a glass fiber filter (1 µm) to prepare FFC water.

Medium powder (Ham's F-10, available from SIGMA) was dissolved in each of the FFC water and untreated DDW, and immediately filtered for sterilization to obtain an FFC-treated medium and an FFC-free medium (control). The media were stored in a refrigerator.

(i) FFC-treated medium (MDM + FFC-treated DDW)

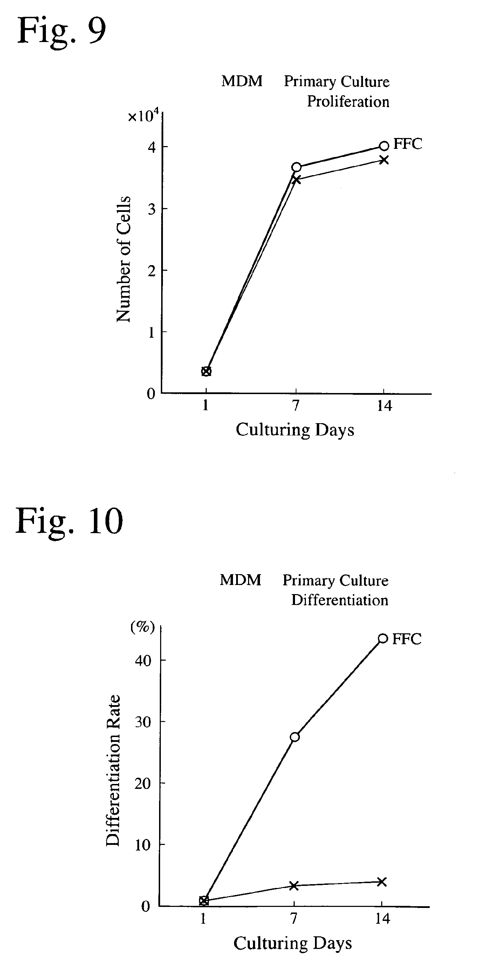

Primary epidermal cells of black mice were cultured in the same manner as in Reference Example 1(i), except for using the FFC-treated medium. The number of cells was measured to determine their differentiation rates. The results are shown in Figs. 9 and 10. The primary culture in the FFC-treated medium resulted in good proliferation of keratinocytes, providing large keratinocyte colonies with increased numbers of keratinocytes. In the FFC-treated medium, keratinocytes became 2-2.5 times as many as in the control. Also, many melanin-forming melanocytes appeared, and when 14 days passed, the percentage of differentiated melanocytes exceeded 40% (Fig. 10). The numbers of melanoblasts and melanocytes were slightly larger in the FFC-treated medium than in the FFC-free medium, though not statistically significant difference (Fig. 9).

(ii) FFC-treated medium + melanocyte-stimulating hormone (MSH)

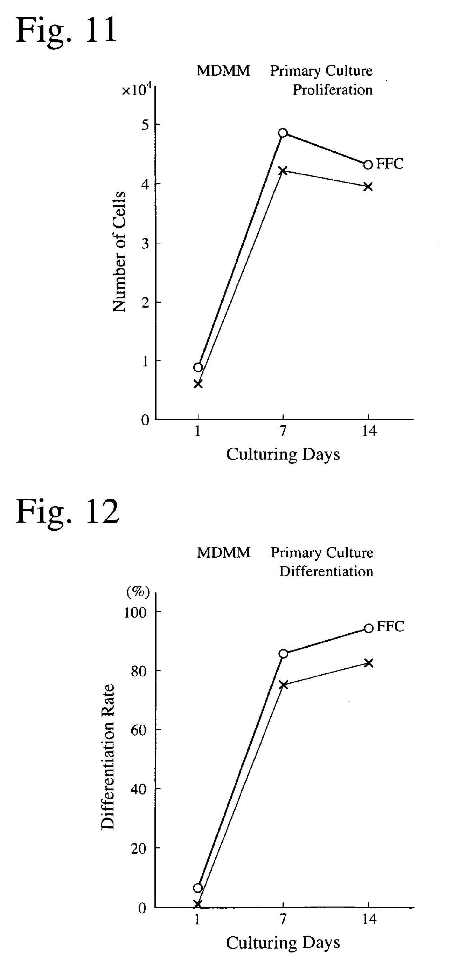

Primary epidermal cells of black mice were cultured in the same manner as in Reference Example 1(i), except for using a medium obtained by adding MSH to the FFC-treated medium. The number of cells was measured to determine their differentiation rates. The results are shown in Figs. 11 and 12. The primary culture in this FFC-treated medium resulted in good proliferation of keratinocytes, providing large keratinocyte colonies with increased numbers of keratinocytes. In this FFC-treated medium, keratinocytes became 2-2.5 as many as in the control, and the proliferation of melanocytes was accelerated, resulting in larger melanocytes and increased melanin and dendrites. The numbers of melanoblasts and melanocytes were slightly larger in this FFC-treated medium than in the FFC-free medium, though not statistically significant difference (Fig. 11).

(iii) FFC-treated medium + dibutyryl cyclic adenosine monophosphate (DBcAMP)

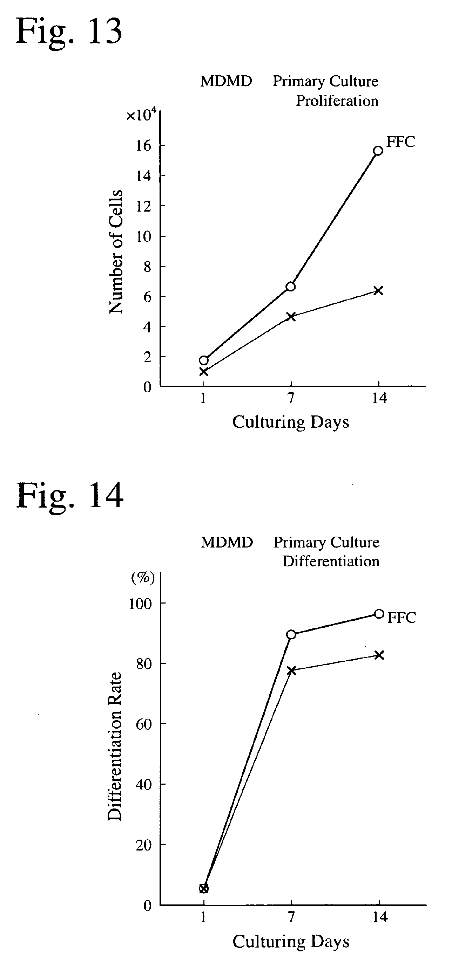

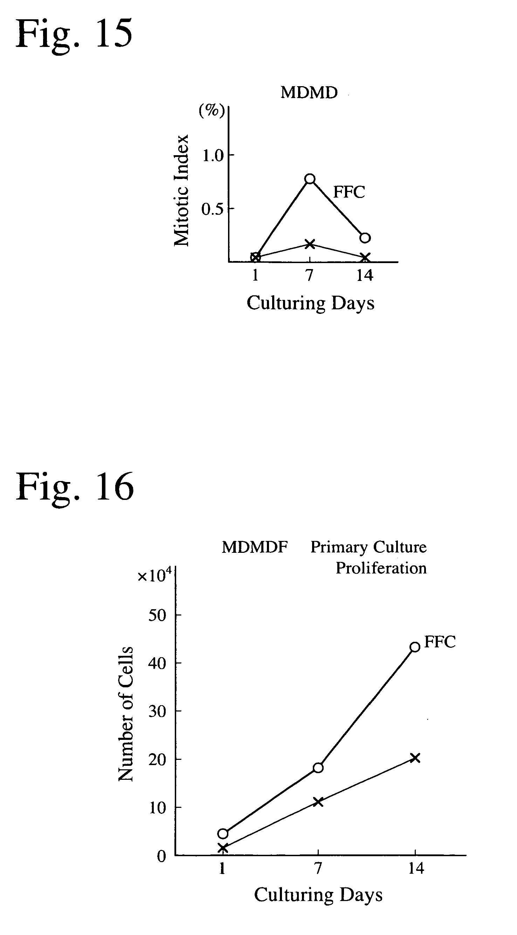

Primary epidermal cells of black mice were cultured in the same manner as in Reference Example 1(i), except for using a medium obtained by adding DBcAMP to the FFC-treated medium. The number of cells was measured to determine their differentiation rates. The results are shown in Figs. 13 and 14. The mitotic index is shown in Fig. 15. The primary culture in this FFC-treated medium resulted in good proliferation of keratinocytes, providing large keratinocyte colonies with increased numbers of keratinocytes. In this FFC-treated medium, keratinocytes became 2-2.5 as many as in the control, and the proliferation of melanocytes was accelerated, resulting in larger melanocytes and increased melanin and dendrites. Cells started proliferating after 3-4 days, and after 14 days, they became about 2.5 times as many as in the control, with high cell division frequency (Fig. 15). It was found that the FFC-containing medium acted synergistically with DBcAMP to let melanocytes proliferate.

(iv) FFC-treated medium + DBcAMP + basic fibroblast growth factor (bFGF)

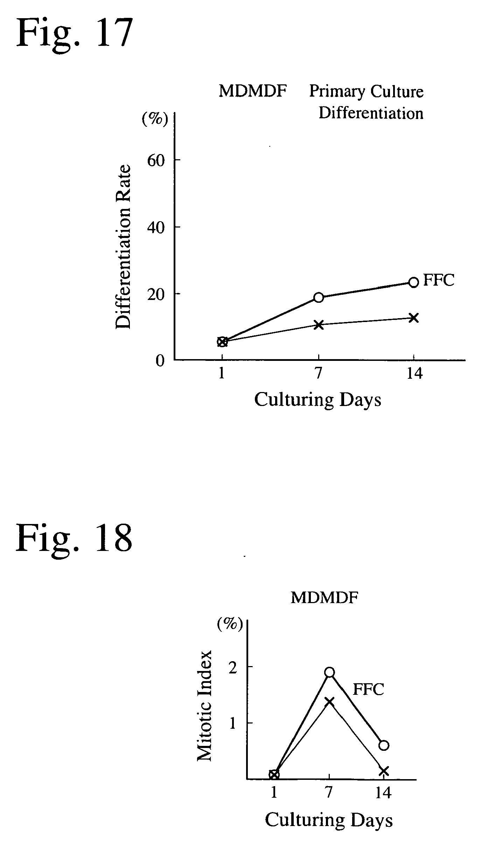

Primary epidermal cells of black mice were cultured in the same manner as in Reference Example 1(i), except for using a medium obtained by adding DBcAMP and bFGF to the FFC-treated medium. The number of cells was measured to determine their differentiation rates. The results are shown in Figs. 16 and 17. The mitotic index is shown in Figs. 15 and 18. The primary culture in this FFC-treated medium resulted in good proliferation of keratinocytes, providing large keratinocyte colonies with increased numbers of keratinocytes. In this FFC-treated medium, keratinocytes became 2-2.5 as many as in the control. Melanoblasts actively started proliferating after 3-4 days from the start of culture, and after 14 days, they became about 2 times as many as in the FFC-free from medium. This FFC-treated medium provided high cell division frequency and high percentage of melanocytes (Fig. 18). It was found that the FFC-containing medium acted synergistically with DBcAMP and bFGF to let melanoblasts proliferate.

The culture of primary epidermal cells of black mice with the FFC-treated medium accelerated the proliferation of keratinocytes, as well as the proliferation and differentiation of melanoblasts and melanocytes. This indicates that the FFC-treated water can keep the homeostasis of skin cells.

Comparative Example 1

A medium containing metals ions eluted from granite porphyry ceramic was obtained in the same manner as in Reference Example 2, except for using about 400 g of granite porphyry ceramic (available from Kisone Bussan) in place of the FFC ceramics. The concentrations of main metals contained in distilled water in which the granite porphyry ceramic was immersed are shown in Table 3.

**Table 3**

| Elements | Concentration (g/L) |
|---|---|
| Potassium | 7.1 |
| Sodium | 8.9 |
| Calcium | 8.8 |
| Magnesium | 5.9 |
| Iron | 0.05 |
| Manganese | 0.03 |
| Strontium | 0.04 |
| Zinc | 0.02 |
| Copper | 0.04 |
| Chromium | 0.02 |
| Selenium | 0.005 |
| Molybdenum | 0.0019 |

(i) Granite porphyry-treated medium + dibutyryl cyclic adenosine monophosphate (DBcAMP)

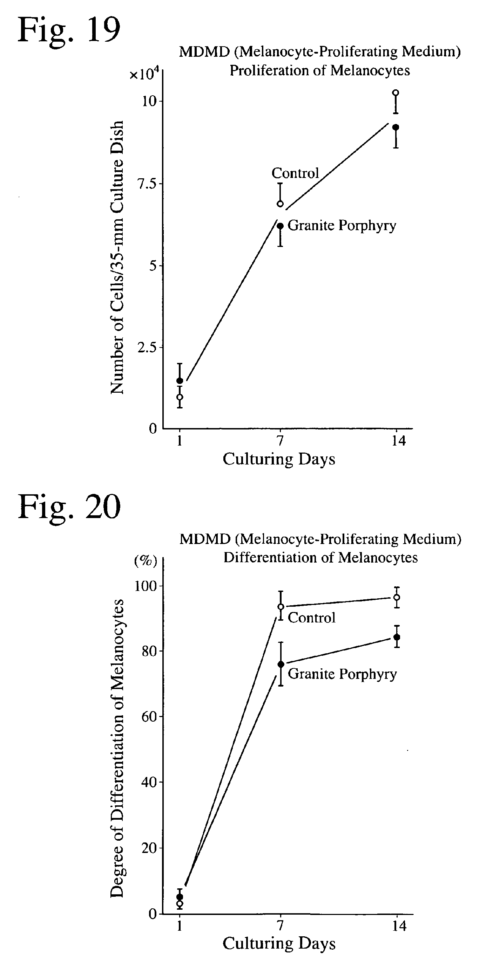

Primary epidermal cells of black mice were cultured in the same manner as in Reference Example 1(i), except for using a medium obtained by adding DBcAMP and bFGF to the granite porphyry-treated medium. The number of keratinocytes in a keratinocyte colony is shown in Table 4. The number and differentiation degree of proliferated melanocytes are shown in Figs. 19 and 20. As is clear from Fig. 19, the culture of primary epidermal cells with this granite porphyry-treated medium suppressed the differentiation of melanocytes, as compared with a medium using granite porphyry-free, distilled water. The number of proliferated cells was substantially the same as in the control.

(ii) Granite porphyry-treated medium + DBcAMP + basic fibroblast growth factor (bFGF)

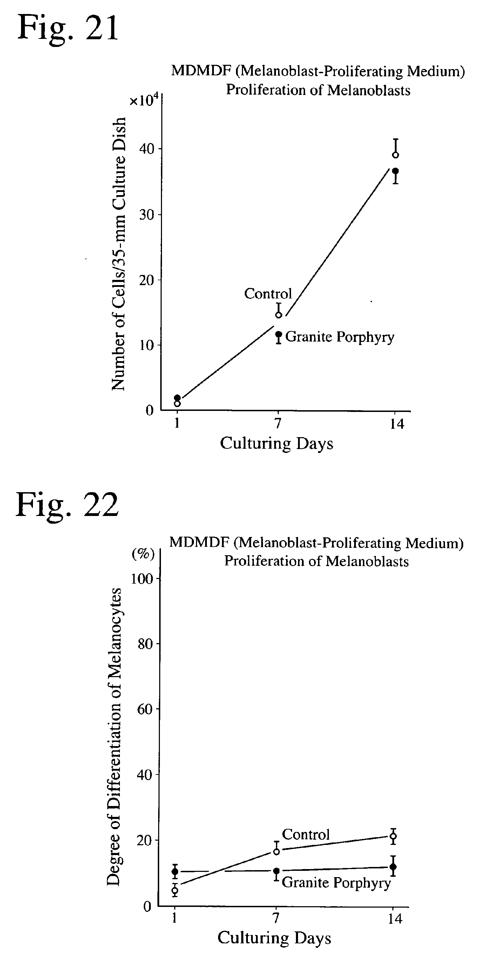

Primary epidermal cells of black mice were cultured in the same manner as in Reference Example 1(i), except for using a medium obtained by adding DBcAMP to the granite porphyry-treated medium. The number of keratinocytes in a keratinocyte colony is shown in Table 4. The number and differentiation degree of proliferated melanocytes are shown in Figs. 21 and 22. As is clear from Fig. 21, the culture of primary epidermal cells with this granite porphyry-treated medium suppressed the differentiation of melanocytes, as compared with a medium using granite porphyry-free, distilled water. The number of proliferated cells was substantially the same as in the control.

**Table 4**

| No. | Culture Medium | Number of Keratinocytes |
|---|---|---|
| - | Untreated Culture Medium + DBcAMP (Control) | 6.86 ± 1.22 |
| Reference Example 2(iii) | FFC-Treated Culture Medium + DBcAMP | 17.57 ± 2.65 |
| Comparative Example 1(i) | Granite Porphyry-Treated Culture Medium + DBcAMP | 15.27 ± 2.77 |
| - | Untreated Culture Medium + DBcAMP + bFGF (Control) | 7.49 ± 1.16 |
| Reference Example 2(iv) | FFC-Treated Culture Medium + DBcAMP + bFGF | 16.63 ± 3.66 |
| Comparative Example 1(ii) | Granite Porphyry-Treated Culture Medium + DBcAMP + bFGF | 15.30 ± 2.64 |

Although the culture of primary epidermal cells of black mice with a granite porphyry-treated medium accelerated the proliferation of keratinocytes, it did not influence the proliferation of melanoblasts and melanocytes. It was found that although the granite porphyry ceramic acted on keratinocytes, it did not activate melanoblasts and melanocytes, failing to keep the homeostasis of epidermal cells. Such difference in the proliferation of melanoblasts and melanocytes, which is shown by Reference Example 2 and Comparative Example 1, is presumably derived from the fact that the FFC-treated water contains a water-soluble dimer iron, while the granite porphyry-treated water does not contain a water-soluble dimer iron.

### EFFECT OF THE PRESENT INVENTION

The cosmetic preparation of the present invention obtained by adding magnesium ion and calcium ion to an aqueous solution of a water-soluble iron salt dimer accelerates the proliferation and differentiation of keratinocytes, melanoblasts and melanocytes, so that it has excellent whitening effects, such as the maintenance of a white and fresh skin, and the prevention and curing of stains and freckles.

## Claims

1. A cosmetic preparation comprising an aqueous solution of a water-soluble iron salt dimer, said aqueous solution containing magnesium ion and calcium ion.

2. The cosmetic preparation according to claim 1, wherein the concentration of said water-soluble iron salt dimer is 0.01-10000 µg/L.

3. The cosmetic preparation according to claim 1 or 2, wherein the concentration of said magnesium ion is 0.01-5000 mg/L.

4. The cosmetic preparation according to any one of claims 1-3, wherein the concentration of said calcium ion is 0.1-10000 mg/L.
